# EUROPEAN PATENT APPLICATION

(11) **EP 4 692 889 A1**
(43) Date of publication of application: **11.02.2026**
(21) Application number: 24193332.4
(22) Date of filing: 07.08.2024
(51) Int. Cl.: G02B 21/00, G02B 21/24, A61B 34/20

(54) **ARTICULATED MICROSCOPE AND METHOD TO OPERATE AN ARTICULATED MICROSCOPE**

(71) Applicant: Leica Instruments (Singapore) Pte. Ltd., Singapore 618299 (SG)
(72) Inventor: Themelis, George, 618299 Singapore (SG)
(74) Representative: Paustian & Partner Patentanwälte mbB

(57) **Abstract**

A first aspect of the present disclosure is related to an articulated microscope with an autofocus,
configured to:
- be mounted in a first position;
- obtain information that the microscope has been moved from the first position to a second position;
- adjust the focus to observe an object based on the information that the microscope has been moved to the second position.

## Description

### Technical Field

This disclosure is related to devices for autofocusing an articulated microscope and to methods for operating articulated microscopes.

### Background

Articulated microscopes can be mounted on an arm or similar positional support systems in order to be flexibly positioned within their workspace and/or share their workspace with other objects or users. An arm-mounted microscope, e.g., may feature an articulating arm that allows for flexible positioning of the microscope head, enabling users to inspect objects from various angles and distances. A microscope mounted on a positional support system may be tilted, rotated, and moved to different positions, thereby providing a wide range of motion for examining objects of different sizes and shapes without needing to move them. This flexibility is particularly beneficial for inspecting large or irregularly shaped specimens. Additionally, an articulated microscope can ensure accurate and consistent observations, making it ideal for applications that require detailed examination, such as biological or medical analyses. Improvements for articulated microscopes are desirable.

### Summary

An object of the present disclosure is to improve working with an articulated microscope.

This object is solved by the disclosed embodiments, which are defined in particular by the subject matter of the independent claims. The dependent claims provide information for further embodiments. Various aspects and embodiments of these aspects are also disclosed in the summary and description below, which provide additional features and advantages.

A first aspect of the present disclosure is related to an articulated microscope with an autofocus,
configured to:
- be mounted in a first position;
- obtain information that the microscope has been moved from the first position to a second position;
- adjust the focus to observe an object based on the information that the microscope has been moved to the second position.

An articulated microscope can be any microscope that has a workspace that exceeds it autofocus range. A workspace is a physical space in which an articulated microscope can be positioned. An autofocus can be configured to adjust a focus until it is accurate. Additionally or alternatively an autofocus can be a configured to adjust a previously adjusted focus and/or a pre-determined focus, which can be obtained, e.g. over a communication means.

An articulated microscope can be mounted or comprise a means in order to be movable within the workspace. For example, an articulated microscope can be mounted on a surgery table via an articulated arm. Further examples are provided in the remainder. An articulated microscope can also be mounted in a fix position. In this case the microscope needs to be related to a movable specimen holder. A specimen holder can be movable in particular be being mounted or comprising one of the means described above. Combinations of a movable microscope and a movable specimen holder may also be possible. In particular the microscope or the specimen holder may then be mounted on or comprise one of the means described above.

An articulated microscope can also be a handheld microscope. Such a microscope may provide improved mobility, suitable for fieldwork and difficult-to-reach inspections.

A position in general can be any information by which a position within a workspace of the microscope can be expressed. For example, a position can be a Cartesian position involving one or more degrees-of-freedom, in particular three translational degrees of freedom, such as x, y, z - coordinates, and/or three rotational degrees of freedom, such as roll, pitch, and yaw. A position can be an absolute position and/or a relative position.

A first position may be a parking position. A parking position can be a position during a task or between different tasks.

A parking position during a task can be a position in which a user has moved the microscope because she/he does not need the microscope for one or more steps of a current task, while the user needs the microscope for one or more other steps of a current task. For example during a surgery, a surgeon might move away the microscope in order to perform a certain part of the surgery without the microscope. In this case the surgeon can move away the microscope in an area of the working space in which the microscope does not disturb the surgeon and/or other personal. Hence, a parking position can be any position within a given workspace of an articulated microscope.

A parking position during different tasks can be any position in which a microscope is moved when it is not used for a longer time period. Such a parking position may be a storage position and/or a position in which the microscope is protected in a certain way. For example, a parking position can be a position at an upper position of a gantry, in particular near a ceiling. A parking position can be a position to which a microscope is moved automatically. During a parking position, a microscope may lose a previously adjusted focus.

A first position may also be a position where the microscope was used. For example, a position of another perspective on an object, a position of another part of an object, or a position of another object. In this type of first position, the microscope may have a different focus adjusted then needed for a second position.

An information that the microscope has been moved from a first position to a second position can be based on a determination that the microscope has come to a halt at a (second) position that is different from the first position. Additionally or alternatively, an information that the microscope has been moved from a first position to a second position can comprise the second position, in particular can be equal to the second position itself.

A second position may be a position in which the microscope is used or is intended to be used. Therefore, a focus will be adjusted based on the second position. The second position can comprise an absolute position. Additionally or alternatively, the second position can comprise a relative position. For example, a second position can comprise translational position information relative to a first parking position and rotational position information comprising absolute values for roll, pitch, and yaw of the microscope. Alternatively, the second position can be entirely expressed in relation to a first position or entirely be expressed as an absolute position of a position system (e.g. a Cartesian coordinate system) that comprises the workspace of the microscope.

A second position can be determined based on a preceding (second) position where a focus had been adjusted. Additionally or alternatively, a second position can be based on a distance to a sample. The sample's position may have been detected by a sensor, e.g. be based on a camera image that is automatically analyzed with respect to the position of the sample. The second position can also be based on a user input. For example, a surgeon can move the microscope to a position from which she/or he wants to analyze a sample and if the microscope has arrived at this position the surgeon presses a button indicating the microscope's arrival at the second position. The microscope can then start the autofocus procedure.

A second position can also comprise a set of positions, e.g. a second position can be a set of positions having the same distance to a reference position, e.g. marked on an object to be observed.

An articulated microscope according to the first aspect of this disclosure can provide a faster autofocus for an object to be observed after the microscope has been displaced from an actual working position or after the microscope has been moved to a further working position, in particular in which different foci were adjusted.

An embodiment of the first aspect of the present disclosure is related to an articulated microscope with an autofocus,
wherein the microscope is mounted on one or more of:
- an arm mount;
- a ceiling mount;
- a table or surgery-bed mount.

An articulated microscope can, e.g. be mounted on an arm. The arm can be a passive (i.e. non-actuated arm) or an active (i.e. motorized arm). By the use of the arm the microscope can be repositioned within the workspace determined by the arm kinematics. An active arm can be, e.g., a robot arm.

An articulated microscope can be repositioned using several methods beyond arm-mounted setups. For example, a microscope can be mounted on a track stand. A track stand may allow horizontal and/or vertical sliding of the microscope along a fixed path, ideal for examining large specimens or multiple items in sequence. In another example, the microscope can be mounted on a boom stand. A boom stand may allow lateral movement with a horizontal arm, which is in particular suited for inspecting objects from various angles.

A microscope can also be mounted or comprise manual stages. Via a manual stage a user can move a microscope relative to a specimen along Cartesian X and Y axes. In another example, the microscope can be mounted on or comprise a tilting stage. A tilting stage may enable angling of the microscope relative to a specimen for multi-perspective observation. In particular this can be beneficial for analyzing 3D objects.

An articulated microscope may also be mounted on or comprise a gantry system. Via a gantry system, the microscope can cover a larger workspace as if the microscope is mounted on a robot arm.

Based on one of these positional support systems, the microscope can be quickly moved out of the way of a surgeon, for example.

An embodiment of the first aspect of the present disclosure is related to an articulated microscope with an autofocus,
wherein the microscope comprises a sensor, in particular a gyroscope, for observing its position; and wherein the second position is obtained based on information from the sensor.

A sensor can measure a position directly or indirectly. Direct position measurements can be based on electric or magnetic measurement principles which are transformed to position information, e.g. by an MR-sensor. Indirect position measurements can observe velocity or acceleration and derivate the measurement one or two times in order to arrive at a position value. Acceleration can be measured by a gyroscope.

Mounting a sensor, e.g. a gyroscope, to a movable microscope enables precise tracking of its position and orientation. Various mounting methods may include direct attachment to a microscope frame and/or attaching a gyroscope to an articulating arm to track its motion. Integration of a sensor, e.g. a gyroscope, with a specimen stage may allow for monitoring any tilting or shifting during observations. Modular sensor platforms may combine gyroscopes with other sensors for comprehensive motion tracking. Wireless sensor modules offer flexibility by eliminating cables, while custom enclosures protect the sensor and facilitate easy mounting. Mounting on a movable base allows for tracking the entire assembly's movement and integrating the sensor with control systems in order to provide real-time feedback for automatic focus adjustments.

Sensor information related to a first and/or a second position can also come from external devices and may then be obtained via a respective communication interface. However, using own sensor information the microscope can effectively use information to determine a first and/or second position.

An embodiment of the first aspect of the present disclosure is related to an articulated microscope with an autofocus,
configured to:
- being attached to an articulated arm; and - obtaining information about the second position from the articulated arm.

An arm can comprise passive and/or active (i.e. motorized) joints. Additionally or alternatively, an arm can comprise a serial and/or a parallel kinematics.

The arm can comprise an angle sensor in one or more of its joints. The angle information can then be transformed to a Cartesian first/second position. Additionally or alternatively, the arm can be configured to measure a Cartesian position directly, in particular on the mechanical interface between arm and microscope. If the microscope comprises an arm, the reference point for the position measurement can also be closer to the lens, in particular located on the microscope or even on the lens.

In case the arm has a redundant kinematics, an additional decision about a position of the one or more redundant joints may performed. In particular this can be done such that the arm is optimally placed, e.g. such that a distance to a person or an object in the workspace of the articulated microscope is optimized.

Based on these examples, a first and/or second position information can be provided with Fastly and with high accuracy.

An embodiment of the first aspect of the present disclosure is related to an articulated microscope with an autofocus,
comprising one or more markers observable by an external position sensing means; and
configured to obtaining information about the second position from the external position sensing means.

To determine a first and/or second position of a microscope various types of markers can be used. These may include optical markers such as one or more QR codes, barcodes, reflective markers (laser-scannable markers), and LED markers. These marker types can provide precise positioning data when scanned or detected by optical systems. Additionally or alternatively magnetic markers can be used to observe a first and/or second position. One or more magnetic markers can be formed as magnetic strips and dots. Additionally or alternatively RFID markers such as RFID tags may be used to enable wireless position tracking. Additionally or alternatively, radio frequency markers, such as beacons and UWB tags utilize radio frequencies for position measurement.

One or more markers may be attached to the microscope and/or to a device on which the microscope is mounted. Additionally or alternatively, one or more markers can also be located on a specimen holder and its position is provided to the microscope.

An advantage of using these markers is that they enable the determination of absolute position related to both the microscope and the object with high accuracy.

An embodiment of the first aspect of the present disclosure is related to an articulated microscope with an autofocus,
configured to:
- determining if a current focus is valid for the second position.

A validation of a focus can be performed before the focus is adjusted or after the focus is adjusted based on the second position.

A focus can be validated by comparing it to a previously adjusted focus. In some embodiments, a focus is assigned to an initial position, which is equal or in a vicinity of the second position and stored for later usage. In a next step, the microscope is moved to the first position, e.g. an short-term parking position during a surgical procedure. The movement to the first position is detected, as the distance to the patient increased a pre-defined threshold. Afterwards, the microscope is moved to the second position in order to be used again by a surgeon. This position should be essentially the same as the initial position and the stored focus of the initial position can be adjusted. However, before the focus of the initial focus is adjusted, it is determined if it is the correct focus. Therefore, the second position is compared to the initial position and the focus for the second position is only validated if the second position is equal or within a pre-defined threshold distance to the initial position.

Additionally or alternatively, a focus can be validated by a user confirmation.

An embodiment of the first aspect of the present disclosure is related to an articulated microscope with an autofocus,
wherein the adjustment based on the second position is a coarse adjustment; and
configured to:
   - adjust the focus independent from the second position.

In particular, when a focus should be adjusted for a second position that is the same or at least similar to a previous position in which a focus was adjusted, a focus adjustment can comprise a coarse adjustment and a fine adjustment.

A coarse adjustment can be implemented by adjusting a previously adjusted focus, in particular the latest adjusted operational focus (wherein an operational focus is a focus used to analyze an object and therefore differs from a focus of a parking position). This coarse focus is adjusted if the microscope has reached a second position (which can be determined by a halt of the microscope that is distant from a parking position). Additionally or alternatively, the coarse focus can be already adjusted if it is detected that the microscope is moved towards an object to be analyzed and/or another reference point.

However, after the coarse focus was adjusted it cannot be assumed that the focus is fully adjusted then, because the object to be observed might have changed/moved and/or the second position might differ, at least slightly, from the position in which the previous focus was obtained. To account for this, in a step after the second-position-based-focus-adjustment, a fine adjustment can be conducted in order to accurately adjust the focus. The fine adjustment is independent from the second position and may only depend on the object to be analyzed.

By these examples, time-to-focus can be reduced to arrive at an accurate focus.

An embodiment of the first aspect of the present disclosure is related to an articulated microscope with an autofocus, wherein the focus is adjusted based on the second position if a velocity of the microscope relative to the object is essentially zero.

A second position can be a position in which the microscope is standing still. This can be in particular a position from which an object should be observed. This embodiment assures that an autofocus is only performed from a position from which an observation of an object is possible (positions at which the microscope still moves - at least with a velocity that exceeds a pre-defined threshold - may not be positions from which an observation can be performed)

An embodiment of the first aspect of the present disclosure is related to an articulated microscope with an autofocus,
wherein the focus is adjusted based on the second position if a time-based derivative of the second position is essentially different from zero.

A second position can be a position, e.g. defined by a distance to an object to be observed, and when the second position is crossed (i.e. with a velocity unequal to zero), a pre-defined autofocus can be adjusted. Then, when the microscope is halted at a position on which the object should be observed, the microscope may already have a (coarsely) adjusted focus. If necessary, a fine focus can then still be performed.

An embodiment of the first aspect of the present disclosure is related to an articulated microscope with an autofocus,
configured to:
- obtain a focus position; and
wherein the focus is adjusted based on the second position and a direction of movement that led to the second position being towards the focus position.

A focus position can be a reference point, e.g. a position of a surgery table or a reference point provided by a user over a GUI. A focus position can be a position in which a microscope analysis should be carried out.

When the microscope arrives at the second position it is evaluated if it came, e.g., from a parking position or if it came from a position in which an operational focus for use of the microscope was adjusted. If it came from a parking position, a focus can be adjusted in order to arrive at least with a decent adjusted focus at an operational position. If the microscope came from an operational position and crosses the second position, it can be assumed that the microscope is moved to a parking position. Then no focus needs to be adjusted.

Based on these examples, an autofocus needs not adjust a focus if the microscope is moved away from the object. This can save unnecessary focusing. The autofocus may only adjust the focus if microscope is moved towards the object. This can reduce a time-to-focus.

An embodiment of the first aspect of the present disclosure is related to an articulated microscope with an autofocus,
configured to:
- obtain a focus position; and
wherein the focus is adjusted based on the second position and if the second position is within a pre-defined threshold distance to the object.

This can be the case, e.g., if a second position is only defined by a stop that is distant from a first position. Then it is checked if the second position is close enough to a focus position (i.e. a reference position as explained above). If this is the case a focus is adjusted by the autofocus system. If this is not the case, no autofocus is performed.

In other words, the autofocus function may only be performed if the second position is close enough to an object to be observed.

An embodiment of the first aspect of the present disclosure is related to an articulated microscope with an autofocus,
wherein the focus is adjusted based on the second position and on the first position, in particular if an Euclidian distance between the first position and the second position exceeds a pre-defined threshold.

If the first position is a parking position, then the autofocus should only start if the distance between the parking position and the second position is large enough.

However, if the first position was a position in which a microscope was used, then an autofocus may be adjusted only if an Euclidian distance between the parking position and the second position is smaller than a predefined threshold. This assures that an autofocus is only performed, in case the microscope has arrived at an approximate location where the microscope analysis should be performed.

An embodiment of the first aspect of the present disclosure is related to an articulated microscope with an autofocus,
configured to:
- determine type of movement of the microscope being either a parking-movement or a use-movement; and
- adjust the focus based on the second movement only if a use-movement is determined.

A determination of movement-type can be done by an machine learning algorithm, e.g. a neuronal network. Then it can be determined if a movement of a microscope is a movement towards a parking position or towards any other position where the microscope is not used. For these positions an autofocus adjustment is not necessary. Additionally or alternatively, it can be determined if a movement of a microscope is towards a position in which a microscope should be used. In this case, the autofocus can already be activated and adjust the best possible focus or a previously adjusted focus, in particular as a coarse focus.

Thereby, the system can automatically determine which movements require a focus adjustment and which do not.

A second aspect is related to a method for controlling an autofocus of a microscope,
comprising the steps:
- obtaining information about a first position of an articulated microscope;
- obtaining information that the microscope has been moved from the first position to a second position;
- determining information for an autofocus of an articulated microscope based on the information that the microscope has been moved to the second position;
- control the focus of the articulated microscope based on the information for the autofocus; and/or provide the information for the autofocus for the articulated microscope.

A method according to the second aspect can comprise one or more steps described in relation to the first aspect. This can be independent of the other steps that are described together with the respective step.

Furthermore, a method according to the second aspect of this disclosure can be executed by a processor in a microscope according to the first aspect of this disclosure. Additionally or alternatively, a method according to a second aspect can be executed by a network device that obtains information from a microscope according to the first aspect of this disclosure and which provides information to control the autofocus of the microscope (therefore, the information for the autofocus can be provided for the articulated microscope). By executing the method on a network device foci of a plurality of microscopes can be adjusted.

A third aspect of the present disclosure is related to a device for controlling an autofocus of a microscope,
configured to:
- obtain information about a first position of an articulated microscope;
- obtain information that the microscope has been moved from the first position to a second position;
- determining information for an autofocus of an articulated microscope based on the information that the microscope has been moved to the second position;
- provide the information for the autofocus for the microscope.

A device according to the second aspect can be a network-based controller. Such a device may comprise one or more structures and/or functions described in relation to the first aspect. Furthermore, such a device is equipped with communication interfaces to communicate with a microscope according to the first aspect of this disclosure.

Such a device can be arranged independently from the microscope and/or may serve a plurality of microscopes.

### Brief description of the figures

Further advantages and features result from the following embodiments, some of which refer to the figures. The figures do not always show the embodiments to scale. The dimensions of the various features may be enlarged or reduced, in particular for clarity of description. For this purpose the figures are at least partially schematized.
Fig. 1 illustrates an articulated microscope system 100 with a passive arm according to embodiments of this disclosure.
Fig. 2 illustrates an articulated microscope system 200 with a target-space-based focus adjustment according to embodiments of this disclosure.
Fig. 3 illustrates an articulated microscope system 300 with a reference-point-based focus adjustment according to embodiments of this disclosure.
Fig. 4 illustrates an articulated microscope system 400 with a gantry according to embodiments of this disclosure.
Fig. 5 illustrates an articulated microscope system 500 according to embodiments of this disclosure.

In the following description reference is made to the accompanying figures which form part of the disclosure, and which illustrate specific aspects in which the present disclosure can be understood. Identical reference signs refer to identical or at least functionally or structurally similar features.

In general, a disclosure of a described method also applies to a corresponding device (or apparatus) for carrying out the method or a corresponding system comprising one or more devices and vice versa. For example, if a specific method step is described, a corresponding device may include a feature to perform the described method step, even if that feature is not explicitly described or represented in the figure. On the other hand, if, for example, a specific device is described on the basis of functional units, a corresponding method may include one or more steps to perform the described functionality, even if such steps are not explicitly described or represented in the figures. Similarly, a system can be provided with corresponding device features or with features to perform a particular method step. The features of the various exemplary aspects and embodiments described above or below may be combined unless expressly stated otherwise.

### Detailed description

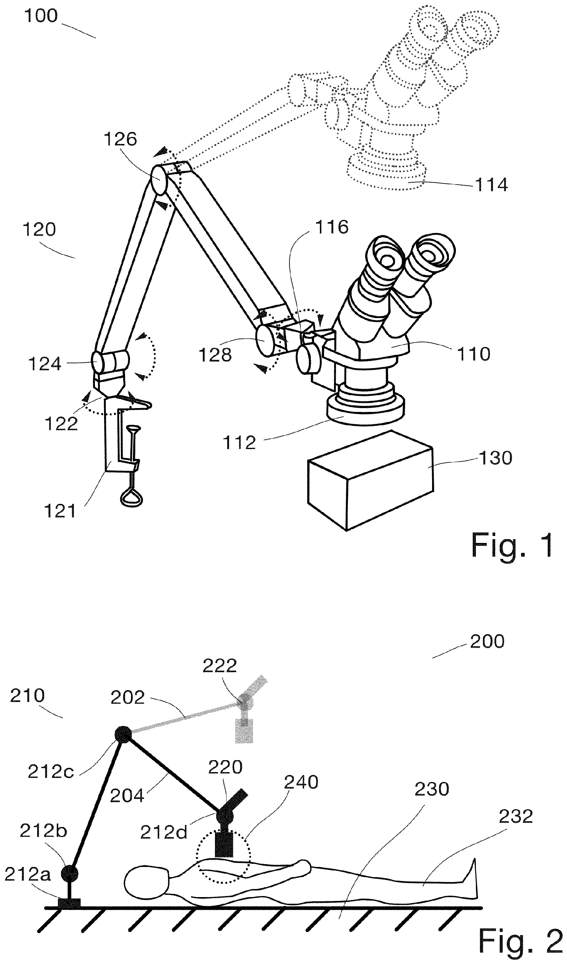

Fig. 1 illustrates an articulated microscope system 100 with a passive arm according to embodiments of this disclosure. The microscope system 100 comprises a microscope 110. The microscope 110 is mounted on an articulated arm 120. The arm 120 is a passive arm with no actuation. Movement of the arm 120 is done by a user, such as a surgeon. The microscope system 100 is configured to observe a specimen 130 from different positions and from different angles.

The microscope 110 comprises a lens system with which the specimen 130 can be observed in a focused position 112. Furthermore, the microscope 110 comprises a rotational joint 116 by which the microscope is coupled to the passive arm 120. The joint 116 provides a rotational degree of freedom around a roll-axis of the microscope.

The passive arm 120 comprises a fastening means 121, such as a screwed-clamp, to mount the microscope system 100 flexibly to a suitable support. Such a support can be, e.g. a surgical table (not depicted). The arm 120 comprises links that are coupled by a plurality of joints. A first joint 122 provides a rotational degree-of-freedom of the system 100 around a yaw-axis. The joint 122 further provides a coupling between the fastening means 121 and the first link of the arm 120. A second joint 124 provides a rotational degree-of-freedom of the system 100 around a first pitch-axis. The joint 124 is directly coupled to the joint 122. A third joint 126 provides a rotational degree-of-freedom of the system 100 around a second pitch-axis. A fourth joint 128 provides a rotational degree-of-freedom of the system 100 around a third pitch-axis.

The microscope system 100 can be positioned in a workspace that is defined by the mounting position, the joints 122, 124, 126, 128, the links between the joints of the arm 120 and by the joint 116 of the microscope 110. Within the workspace the microscope 110 can be arranged flexibly in order to observe the specimen 130. All joints are equipped with position sensors. Based on the position information, the microscope exactly knows at which position it is. Additionally or alternatively, the system may be equipped with a gyroscope or a force sensor to determine position and/or motion information.

When the microscope is not used, e.g., when the user wants to view the specimen directly (i.e. without the microscope), the microscope 110 can be moved to a position where the microscope does not obstruct the user and/or where the microscope is safe. Such a position illustrated by the parking position 114 (dotted part of microscope system 100). The parking position maybe regarded as a first position of the articulated microscope system 100.

During an analysis of a specimen (e.g. of a patient's organ during a surgery), there can be several phases during which the microscope is used and several phases during which the microscope is not used. If the microscope is not used, it can be moved to a parking position.

When the microscope is moved to a parking position 114 the autofocus may change the focus of the microscope. Then, if the microscope is moved back to the working position 112 the autofocus has to re-adjust the focus, this may take time. One reason for this is that the autofocus does not know where the microscope is in relation to the specimen. In this case, the autofocus may have to search a focus essentially based on a trial-and-error method, by changing the autofocus in arbitrary directions and analyze a result.

In order to work efficiently a focus of the microscope should be adjusted as fast as possible. Therefore, the focus can be readjusted depending on the working position 112, which can be a second position in terms of the first or the second aspect of the disclosure as described above.

For example, if the microscope system is moved from a parking position 114 to a working position 112 it can be analyzed if the working position is closer to the specimen (e.g. operationalized by a position of a specimen holder) than a previously taken working position. Based on this information, the autofocus knows in which direction the focus must be adapted in order to reach an accurate focus. This can be implemented as a function of the microscope or of a device that controls the autofocus of the microscope.

In another example, the microscope is moved from a parking position 114 (first position) to another position (second position). If the system understands that the system is approaching the specimen, the autofocus of the microscope can be activated, because it infers that the microscope is moved to a working position 112. This can be implemented as another function alternatively or additionally to the previously described function. Advantageously, by this function, the autofocus needs not to be always active to focus the microscope. If the system understands from its position information that the microscope is not moved to a working position 112, but, e.g., only moved to another parking position 114 (because the old parking position might be used for other purposes), it will not activate the autofocus.

Fig. 2 illustrates an articulated microscope system 200 with a target-space-based focus adjustment according to embodiments of this disclosure. The microscope system can be a microscope system 100 according to Fig. 1. The microscope system 200 comprises an articulated arm 210, e.g. a passive arm or an active robot arm. The arm comprises four joints 212a, 212b, 212c, 212d to adjust yaw and pitch of the microscope 220. The microscope 220 is mounted on the tip of the arm 210 and includes a joint 222 to control the roll-axis of the microscope.

In this case, the microscope is used during a surgery. The microscope system 200 is mounted on a patient table 230 to analyze an organ of a patient 232 during the surgery.

In order to use the microscope during the surgery flexibly, the microscope can be moved away from a working position 204 into a parking position 202. The parking position (indicated in grey) can be a pre-defined position to which the microscope system 200 is automatically moved. This requires an active articulated arm 210, e.g. a robot arm. Additionally or alternatively, the system can also comprise further automating parking positions. That enables a flexible use of the working space. Additionally or alternatively, the system can comprise a mode in which a user can move the system to a parking position. This can be an arbitrary parking position which does not to be related to the one or more parking positions for automated parking.

The system of this embodiment further knows where the working position 204 is approximately. This can be known to the system due to previously taken working positions, in particular by machine learning or by statistical inference. Additionally or alternatively, the user might have provided this information via a user interface. In this context the system also knows a focus configuration at the working position. The focus position can also be determined by machine learning or a statistical inference. Additionally or alternatively, a focus configuration can be recorded as a focus that was adjusted when the user provided information about a working position. Depending on the working position the system can determine when the user moves the microscope from a parking position 202 (first position) to the working position 204 (second position).

Furthermore, the system can determine a predefined distance to the working position. This distance is illustrated by the target sphere 240 (illustrated as a circle in the two-dimensional figure). The system starts adjusting a focus assigned to the working position (as described in the previous paragraph) when the system has crossed the sphere/circle 240 towards the working position 204. Then autofocus has already adjusted a focus for the working position 204, at least coarsely, when the microscope arrives at the working position 204. Depending on the accuracy of the focus, the autofocus can still perform a fine tuning of the focus once the microscope has arrived at the working position. This is in particular advantageous if the working position does not exactly equal a previous working position. In any case, a faster focus can be expected.

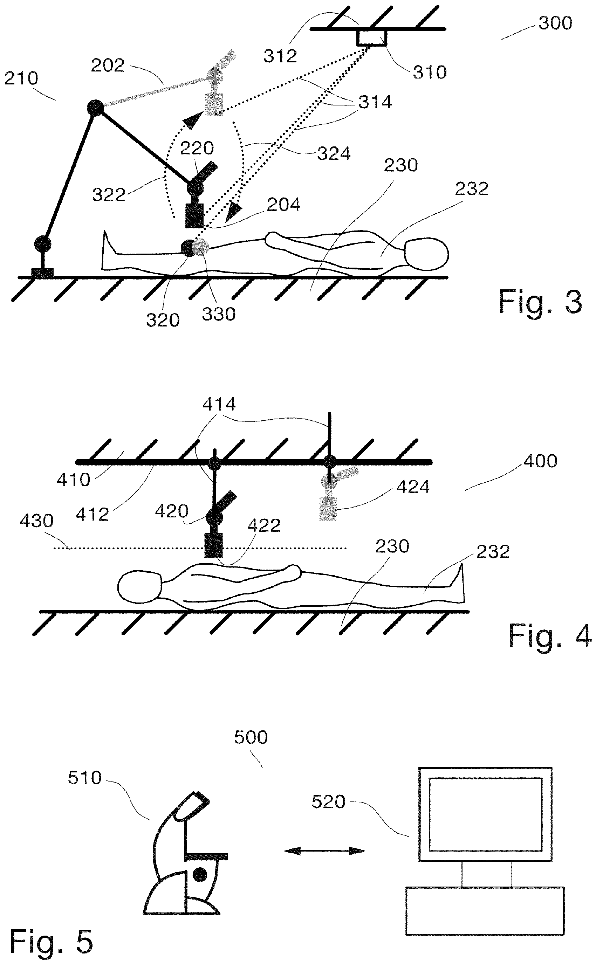

Fig. 3 illustrates an articulated microscope system 300 with a reference-point-based focus adjustment according to embodiments of this disclosure. The microscope system 300 can be a microscope system 100 according to Fig. 1 or a microscope system 200 according to Fig. 2. The arm 210 of the microscope 300 system can be an passive or an active arm. A microscope 220 is attached to the tip of the arm 210. The microscope system is configured to operate during a surgery to analyze a patient 232 on a patient table 230. The microscope system 300 can be place in a plurality of parking positions 202 and in a plurality of working positions 204 withing its workspace.

The focus-relevant position of the microscope 220 is determined by a laser scanner 310 mounted on the ceiling 312 of the operation room. The laser scanner is configured to track markers (not depicted) located on the microscope 220, e.g. on the lens and/or on a top of the microscope frame. Therefore, the laser scanner and the markers are arranged such that the focus-relevant position can be determined in a parking position and in a working position by a laser beam 314 beam.

One or more working positions 320 are determined by the location on which the surgery or at least the microscopic observation should take place. This position can also be indicated by markers that can be tracked by the laser scanner using a laser beam 314. The information of the laser beam is communicated to the microscope 220 and/or to a device that controls the microscope, and which may also execute a method according to the second aspect of the invention.

Based on the information about the working position 320, it can be determined if the microscope 220 is located at or moved towards a parking position 202. This can be done by understanding that the microscope moves or has moved in a direction 322 away from the working position 320. In such a phase the autofocus can be deactivated and the lens of the microscope can be protected, such that is not contaminated.

Furthermore, based on the information about the working position 320, it can be determined if the microscope 220 is located at or moved towards the working position 320. This can be done by understanding that the microscope moves or has moved in a direction 324 away from the working position 320. In such a phase the autofocus can be activated and a predetermined and/or previously adjusted focus can be tuned in by the autofocus system. In this case, the autofocus is only used when needed and a time to focus the microscope after being moved to a parking position (and losing its focus) 202 can be reduced.

In another non depicted embodiment, which can be combined with other embodiments of this disclosure, the microscope 320 does not approach the working position 320 from a parking position, but from another working position 330. This can be e.g. another perspective on a surgical cavity. The second working position 330 can be determined in the same way as the first working position 320. As depicted the two working positions can be related to different perspectives of a human knee that is subject to a surgery.

Then the surgeon can switch between the two different working positions and for each working positions 320, 330, a respective predefined focus configuration can be realized. The respective predefined focus configuration can be based on previously adjusted focus configurations and/or on machine learning or statistical inference.

Fig. 4 illustrates an articulated microscope system 400 with a gantry 410 according to embodiments of this disclosure. The gantry comprises a horizontal movement means 412 and a vertical movement means 414. Thereby, the mounted microscope 420 can be moved to working positions 422 and parking positions 424 (indicated in grey).

Based on a predefined distance to a surgery table 230 the system knows if the microscope 420 is moved towards a working position 422 or to a parking position 424. For each possible position of the microscope relative to the surgery table, the position of the microscope that yields the predefined distance to the surgery table is indicated by the plane 430 (illustrated as a dotted line in the two-dimensional figure).

In case, the microscope 420 is moved to a working position and crosses the plane/line 430, he autofocus can be activated and a predefined focus can be adjusted. On the other hand, if the microscope moves away from the patient 232 and crosses the plane/line 430 in the other direction, the autofocus can be turned off.

An idea of the described embodiments of this disclosure is the integration of a position sensing mechanism, such as a gyroscope, sensor-equipped arm, or a stereotactic camera, into an autofocus system for a microscope. This features enables real-time adjustment of focus based on the movement or repositioning of the microscope. By estimating the positional changes and pre-adjusting the focus accordingly, the embodiments can minimize the need for manual fine-tuning once the microscope comes to rest.

Such a embodiments can result in further advantages, such as:
1. Reducing manual intervention: By using position sensing to assist autofocus, the need for manual focus adjustments during microscope repositioning can be significantly reduced, leading to fewer disruptions during procedures.
2. Providing real-time adjustment: The described systems can offer a real-time adjustment of the focus as the microscope moves, thus may improve efficiency and speed of focusing, especially important during complex surgical procedures.
3. Enhancing accuracy: The described system may provide accurate focus adjustment based on precise positional data, reducing the chances of user error and potential inaccuracies in focusing.

Some embodiments relate to a microscope comprising a system as described in connection with one or more of the Figs. 1 to 4. Alternatively, a microscope may be part of or connected to a system as described in connection with one or more of the Figs. 1 to 4.

Fig. 4 shows a schematic illustration of a system 500 configured to perform a method described herein. The system 500 comprises a microscope 510 and a computer system 520. The microscope 510 is configured to take images and is connected to the computer system 520. The computer system 520 is configured to execute at least a part of a method described herein. The computer system 520 may be configured to execute a machine learning algorithm. The computer system 520 and microscope 510 may be separate entities but can also be integrated together in one common housing. The computer system 520 may be part of a central processing system of the microscope 510 and/or the computer system 520 may be part of a subcomponent of the microscope 510, such as a sensor, an actor, a camera or an illumination unit, etc. of the microscope 510.

The computer system 520 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 520 may comprise any circuit or combination of circuits. In one embodiment, the computer system 520 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 520 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 520 may include one or more storage devices, which may include one or more memory elements suitable to the particular application, such as a main memory in the form of random-access memory (RAM), one or more hard drives, and/or one or more drives that handle removable media such as compact disks (CD), flash memory cards, digital video disk (DVD), and the like. The computer system 520 may also include a display device, one or more speakers, and a keyboard and/or controller, which can include a mouse, trackball, touch screen, voice-recognition device, or any other device that permits a system user to input information into and receive information from the computer system 520.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Embodiments may be based on using a machine-learning model or machine-learning algorithm. Machine learning may refer to algorithms and statistical models that computer systems may use to perform a specific task without using explicit instructions, instead relying on models and inference. For example, in machine-learning, instead of a rule-based transformation of data, a transformation of data may be used, that is inferred from an analysis of historical and/or training data. For example, the content of images may be analyzed using a machine-learning model or using a machine-learning algorithm. In order for the machine-learning model to analyze the content of an image, the machine-learning model may be trained using training images as input and training content information as output. By training the machine-learning model with a large number of training images and/or training sequences (e.g. words or sentences) and associated training content information (e.g. labels or annotations), the machine-learning model "learns" to recognize the content of the images, so the content of images that are not included in the training data can be recognized using the machine-learning model. The same principle may be used for other kinds of sensor data as well: By training a machine-learning model using training sensor data and a desired output, the machine-learning model "learns" a transformation between the sensor data and the output, which can be used to provide an output based on non-training sensor data provided to the machine-learning model. The provided data (e.g. sensor data, meta data and/or image data) may be preprocessed to obtain a feature vector, which is used as input to the machine-learning model.

Machine-learning models may be trained using training input data. The examples specified above use a training method called "supervised learning". In supervised learning, the machine-learning model is trained using a plurality of training samples, wherein each sample may comprise a plurality of input data values, and a plurality of desired output values, i.e. each training sample is associated with a desired output value. By specifying both training samples and desired output values, the machine-learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g. a classification algorithm, a regression algorithm or a similarity learning algorithm. Classification algorithms may be used when the outputs are restricted to a limited set of values (categorical variables), i.e. the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine-learning model. In unsupervised learning, (only) input data might be supplied, and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters.

Reinforcement learning is a third group of machine-learning algorithms. In other words, reinforcement learning may be used to train the machine-learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine-learning algorithms. For example, feature learning may be used. In other words, the machine-learning model may at least partially be trained using feature learning, and/or the machine-learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e. outlier detection) may be used, which is aimed at providing an identification of input values that raise suspicions by differing significantly from the majority of input or training data. In other words, the machine-learning model may at least partially be trained using anomaly detection, and/or the machine-learning algorithm may comprise an anomaly detection component.

In some examples, the machine-learning algorithm may use a decision tree as a predictive model. In other words, the machine-learning model may be based on a decision tree. In a decision tree, observations about an item (e.g. a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine-learning algorithms. In other words, the machine-learning model may be based on one or more association rules. Association rules are created by identifying relationships between variables in large amounts of data. The machine-learning algorithm may identify and/or utilize one or more relational rules that represent the knowledge that is derived from the data. The rules may e.g. be used to store, manipulate or apply the knowledge.

Machine-learning algorithms are usually based on a machine-learning model. In other words, the term "machine-learning algorithm" may denote a set of instructions that may be used to create, train or use a machine-learning model. The term "machine-learning model" may denote a data structure and/or set of rules that represents the learned knowledge (e.g. based on the training performed by the machine-learning algorithm). In embodiments, the usage of a machine-learning algorithm may imply the usage of an underlying machine-learning model (or of a plurality of underlying machine-learning models). The usage of a machine-learning model may imply that the machine-learning model and/or the data structure/set of rules that is the machine-learning model is trained by a machine-learning algorithm.

For example, the machine-learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g. of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e. to achieve a desired output for a given input.

Alternatively, the machine-learning model may be a support vector machine, a random forest model or a gradient boosting model. Support vector machines (i.e. support vector networks) are supervised learning models with associated learning algorithms that may be used to analyze data (e.g. in classification or regression analysis). Support vector machines may be trained by providing an input with a plurality of training input values that belong to one of two categories. The support vector machine may be trained to assign a new input value to one of the two categories. Alternatively, the machine-learning model may be a Bayesian network, which is a probabilistic directed acyclic graphical model. A Bayesian network may represent a set of random variables and their conditional dependencies using a directed acyclic graph. Alternatively, the machine-learning model may be based on a genetic algorithm, which is a search algorithm and heuristic technique that mimics the process of natural selection.

### List of reference signs

- 100: articulated microscope system
- 110: microscope
- 112: focused lens system
- 114: parking position
- 116: roll joint
- 120: passive arm
- 121: clamp
- 122: yaw joint
- 124: first pitch joint
- 126: second pitch joint
- 128: third pitch joint
- 130: specimen
- 200: microscope system
- 202: first position
- 204: working position
- 210: articulated arm
- 212a: yaw joint
- 212b: pitch joint
- 212c: pitch joint
- 212d: pitch joint
- 220: microscope
- 222: roll joint
- 230: patient table
- 232: patient
- 240: target sphere
- 300: microscope system
- 310: laser scanner
- 312: ceiling of operating room
- 314: laser
- 320: working position
- 322: direction away from working position
- 324: direction towards working position
- 330: second working position
- 410: gantry
- 412: horizontal movement means
- 414: vertical movement means
- 420: microscope
- 422: working position
- 424: parking position
- 430: pre-defined distance to surgery table
- 500: microscope system
- 510: microscope
- 520: computer system

## Claims

1. An articulated microscope (110) with an autofocus,
configured to:
- be mounted in a first position (102);
- obtain information that the microscope has been moved from the first position to a second position (106);
- adjust the focus to observe an object (104) based on the information that the microscope has been moved to the second position.

2. The microscope according to the preceding claim,
wherein the microscope is mounted on one or more of:
- an arm mount (120);
- a ceiling mount;
- a table or surgery-bed mount (120).

3. The microscope according to the preceding claim,
wherein the microscope comprises a sensor, in particular a gyroscope, for observing its position; and
wherein the second position is obtained based on information from the sensor.

4. The microscope according to one of the preceding claims,
configured to:
- being attached to an articulated arm (120); and
- obtaining information about the second position from the articulated arm (122, 124, 126, 128).

5. The microscope according to one of the preceding claims,
comprising one or more markers observable by an external position sensing means (310); and
configured to obtaining information about the second position from the external position sensing means.

6. The microscope according to one of the preceding claims,
configured to:
- determining if a current focus is valid for the second position.

7. The microscope according to one of the preceding claims,
wherein the adjustment based on the second position (240) is a coarse adjustment; and
configured to:
- adjust the focus independent from the second position.

8. The microscope according to one of the preceding claims,
wherein the focus is adjusted based on the second position (204) if a velocity of the microscope relative to the object is essentially zero.

9. The microscope according to one of the preceding claims,
wherein the focus is adjusted based on the second position (204) if a time-based derivative of the second position is essentially different from zero.

10. The microscope according to one of the preceding claims,
configured to:
- obtain a focus position; and
wherein the focus is adjusted based on the second position (204) and a direction of movement that led to the second position being towards the focus position.

11. The microscope according to one of the preceding claims,
configured to:
- obtain a focus position; and
wherein the focus is adjusted based on the second position and if the second position is within a pre-defined threshold distance (430) to the object.

12. The microscope according to one of the preceding claims,
wherein the focus is adjusted based on the second position and on the first position, in particular if an Euclidian distance between the first position and the second position exceeds a pre-defined threshold.

13. The microscope according to one of the preceding claims,
configured to:
- determine type of movement of the microscope being either a parking-movement or a use-movement; and
- adjust the focus based on the second movement only if a use-movement is determined.

14. A method for controlling an autofocus of an articulated microscope, comprising the steps:
- obtaining information about a first position of an articulated microscope;
- obtaining information that the microscope has been moved from the first position to a second position;
- determining information for an autofocus of an articulated microscope based on the information that the microscope has been moved to the second position;
- control the focus of the articulated microscope based on the information for the autofocus; and/or provide the information for the autofocus for the articulated microscope.

15. A device for controlling an autofocus of an articulated microscope, configured to:
- obtain information about a first position of an articulated microscope;
- obtain information that the microscope has been moved from the first position to a second position;
- determining information for an autofocus of an articulated microscope based on the information that the microscope has been moved to the second position;
- provide the information for the autofocus for the microscope.
